# EUROPÄISCHE PATENTSCHRIFT

(11) **EP 1 044 018 B1**
(45) Veröffentlichungstag und Bekanntmachung des Hinweises auf die Patenterteilung: **22.07.2009**
(21) Anmeldenummer: 98966819.9
(22) Anmeldetag: 30.12.1998
(51) Int. Cl.: A61K 39/00, A61P 35/00

(54) **TUMORVAKZINE FÜR MUC1-POSITIVE KARZINOME**
TUMOUR VACCINES FOR MUC1-POSITIVE CARCINOMAS
VACCIN ANTITUMORAL POUR CARCINOMES MUC1-POSITIFS

(30) Priorität: 30.12.1997 DE 19758400
(43) Veröffentlichungstag der Anmeldung: 18.10.2000
(73) Patentinhaber: MAX-DELBRÜCK-CENTRUM FÜR MOLEKULARE MEDIZIN, 13125 Berlin (DE)
(72) Erfinder: KARSTEN, Uwe, D-10407 Berlin (DE); HANISCH, Franz-Georg, D-50679 Köln (DE); PAULSEN, Hans, D-22399 Hamburg (DE)
(74) Vertreter: Baumbach, Friedrich
(86) Internationale Anmeldenummer: PCT/DE1998/003819
(87) Internationale Veröffentlichungsnummer: WO 1999/034824

(56) Entgegenhaltungen:
- WO-A-88/05054
- WO-A-90/05142
- F-Y. DUPRADEAU ET AL.: "Solid-phase synthesis and immunoreactivity of penta-O-(N-acetyl-alpha-D-galactosaminyl)- MUC1 eicosapeptide, a glycosylated counter part of the highly immunogenic tandem repeat sequence of carcinoma-associated mucin." BIOORGANIC & MEDICINAL CHEMISTRY LETTERS, Bd. 4, Nr. 15, 1994, Seiten 1813-1818, XP002112481 Oxford, Grossbritannien
- J. TAYLOR-PAPADIMITRIOU ET AL.: "Exploiting altered glycosylation patterns in cancer: progress and challenges in diagnosis and therapy." TRENDS IN BIOTECHNOLOGY, Bd. 12, Juni 1994 (1994-06), Seiten 227-233, XP002075467 Amsterdam, Niederlande
- M. PRICE ET AL.: "Summary report on the ISOBM TD-4 workshop: Analysis of 56 monoclonal antibodies against the MUC1 mucin." TUMOR BIOLOGY, Bd. 19, Nr. suppl. 1, Dezember 1997 (1997-12), Seiten 1-20, XP002112482 Basel, Schweiz
- D. SPENCER ET AL.: "Effect of glycosylation of a synthetic MUC1 mucin-core-related peptide on recognition by anti-mucin antibodies." CANCER LETTERS, Bd. 100, Nr. 1-2, 27. Februar 1996 (1996-02-27), Seiten 11-15, XP002112483 Shannon, Irland
- S. GOLETZ ET AL.: "A sequencing strategy for the localization of O-glycosylation sites of MUC1 tandem repeats by PSD-MALDI mass spectrometry." GLYCOBIOLOGY, Bd. 7, Nr. 7, Oktober 1997 (1997-10), Seiten 881-896, XP002112484 Oxford, Grossbritannien
- X. LIU ET AL.: "Structurally defined synthetic cancer vaccines: analysis of structure, glycosylation and recognition of cancer associated mucin, MUC-1 derived peptides. " GLYCOCONJUGATE JOURNAL, Bd. 12, Nr. 5, Oktober 1995 (1995-10), Seiten 607-617, XP002112485 London, Grossbritannien
- U. KARSTEN ET AL.: "Enhanced binding of antibodies to the DTR motif of MUC1 tandem repeat peptide is mediated by site-specific glycosylation." CANCER RESEARCH, Bd. 58, Nr. 12, 15. Juni 1998 (1998-06-15), Seiten 2541-2549, XP002112486 Baltimore, MD, VSA

## Beschreibung

Die Erfindung betrifft neuartige Tumorvakzinen auf der Grundlage der Molekülstruktur des menschlichen epithelialen Muzins (MUC1). Anwendungsgebiet der Erfindung ist die Immuntherapie von Karzinomen.

Epitheliale Muzine sind Glykoproteine mit repetitiven Aminosäuresequenzen und einem hohen Kohlenhydratanteil, die teils membrangebunden sind, teils sezerniert werden und auf vielen Drüsenepithelien vorkommen. Das am besten bekannte epitheliale Muzin ist das membrangebundene MUC1, auch als PEM, PUM, EMA, MAM-6, PAS-O oder Episialin beschrieben (Finn, O., et al., Immunol.Reviews 145:61, 1995), dessen extrazellulärer Teil aus einer variablen Anzahl sich wiederholender Einheiten aus 20 Aminosäuren besteht, den sogenannten "Tandem-Repeats". Das MUC1 ist an sich kein tumorspezifisches Molekül; seine Eignung als Tumorantigen beruht darauf, daß sein Kohlenhydratanteil bei Tumoren qualitativ und quantitativ verändert ist (Burchell,J., und Taylor-Papadimitriou,J., Epith.Cell Biol. 2:155, 1993). Dabei treten neue Epitope auf, die vom Immunsystem (humorale und zelluläre Abwehr) wahrgenommen werden.
Nach operativer Entfernung des Primärtumors (bzw. nach Strahlen- oder Chemotherapie) muß in der Regel davon ausgegangen werden, daß noch Tumorzellen im Körper verbleiben ("minimal residual disease"). Diese Tumorzellen, die eine potentielle Gefahr darstellen, werden durch verschiedene körpereigene Mechanismen bekämpft, deren Wirksamkeit durch eine adjuvante Immuntherapie verstärkt werden kann. Die effektivste adjuvante Immuntherapie ist die Vakzinierung. Zwei Voraussetzungen sind hierfür erforderlich: erstens, daß ein geeignetes Zielantigen (Epitop) auf den Tumorzellen vorhanden ist, und zweitens, daß es gelingt, eine möglichst stark immunogene, vorzugsweise synthetische Form einer Vakzine herzustellen.

Nicht-glykosylierte Oligo-Repeat-Peptide des MUC1 stellen ein geeignetes Zielantigen bei einer Reihe häufiger Karzinome dar (Apostolopoulos, V., und McKenzie,I.F.C., Crit.Rev.Immunol. 14:293, 1994). Die immundominante Region des MUC1 ist das Motiv PDTRPAP, das auf jedem Tandem-Repeat vorhanden ist. Bisherige Versuche, eine Vakzine auf der Basis eines einzelnen Tandem-Repeats zu entwickeln, waren jedoch nicht erfolgreich. Nach dem bisherigen Stand des Wissens ist für das Zustandekommen der immunogenen Konformation des Peptids eine Mindestlänge des Peptids erforderlich, die erst bei 3-5 Tandem-Repeats erreicht wird (Fontenot,J.D., et al., J.Biomol.Struct.Dyn. 13:245, 1995). Liu, X. et al. offenbart ein MUC + Peptid (GVTSAPDTRPAPGSTA) mit erhöhter Antigenität, glycosyliert am Threonin 3 oder/und Serin 4, sowie Tumorvakzine .(Glycoconjungate Journal, 12: 607, 1995).

Der Erfindung liegt die Aufgabe zugrunde, auf der Basis der Molekülstruktur des menschlichen epithelialen Mucins MUC1 eine Tumorvakzine zu entwickeln, die insbesondere zur Bekämpfung von nach anderen Therapien im Körper noch verbliebenen Tumorzellen geeignet ist.

Bei der immunologischen Untersuchung synthetischer Glykopeptide, die einem Tandem-Repeat des MUC1 entsprechen, wurde überraschend gefunden, daß die Glykosylierung des Threonins innerhalb der immundominanten Region PDTRPAP mit α-GalNAc die Antigenität signifikant erhöht. Bisher war davon ausgegangen worden, daß diese Position bei nativem MUC1 nicht glykosyliert ist. Zu dieser Schlußfolgerung hatten die Annahme, daß eine Glykosylierung die Erkennung von Peptidepitopen in der Regel behindert, sowie Ergebnisse von *in-vitro-*Glykosylierungsversuchen (Stadie, T., et al., Eur.J.Biochem. 229: 140(1995)) geführt. Neueste Untersuchungen (Müller,S., et al., J.Biol.Chem. 272:24780, 1997) zeigen allerdings, daß das Threonin im PDTRPAP-Motiv *in vivo* sehr wohl glykosyliert sein kann. Aus den genannten neuen Ergebnissen wird geschlossen, daß die Antigenität (und im Zusammenhang damit auch die Immunogenität) des MUC1-Tandem-Repeats durch Glykosylierung des Thr im PDTRPAP-Motiv mittels GalNAc oder einem kurzen Oligosaccharid erheblich erhöht wird. Dadurch wird die immunogene Konformation der immundominanten Region bereits von einem einzelnen Tandem-Repeat erreicht. Die Antigenität des glykosylierten PDTRPAP-Motivs in einem Monorepeat übertrifft sogar die des oligomeren, nicht glykosylierten Peptids.
Auf der Basis dieser Entdeckung wird vorgeschlagen, Tumorvakzinen verschiedener Molekülgrößen zu entwickeln, die am Thr des PDTRPAP-Motivs durch GalNAc oder ein geeignetes kurzkettiges Oligosaccharid glykosyliert sind. Die Aufgabe der Erfindung wird gemäß Anspruch 1 gelöst, die Unteransprüche sind Vorzugsvarianten.

Die Erfindung soll nachstehend durch ein Ausführungsbeispiel näher erläutert werden.

### Beispiel 1

### Antigenität von synthetischen, MUC1-abgeleiteten Glykopeptiden

Im folgenden wird die Bindung von monoklonalen Antikörpern gegen das immundominante Motiv PDTRPAP des epithelialen Muzins (MUC1) an synthetische Glykopeptide dieses Muzins mit einer Länge von 20 bzw. 21 Aminosäuren in einem Festphasen-Immunoassay (ELISA) untersucht. Die Glykopeptide mit den Bezeichnungen A1 bis A12 sind in Tabelle 1 aufgeführt. Sie entsprechen einem überlappenden Tandem-Repeat des MUC1 und enthalten 5 potentielle Glykosylierungsstellen (3x Thr, 2x Ser); A1-A9 enthalten ein zusätzliches Ala. Die Glykopeptide unterscheiden sich in der Zahl und Position der Glykosylierungsstellen (siehe Tabelle 1). A1-A9 tragen als Glykane das Thomsen-Friedenreich-Antigen (TF) β-D-Gal(1-3)a-D-GalNAc-O-*R*, während All und A12 lediglich a-D-GalNAc-O-R (das Tn-Antigen) tragen. Die benutzten Antikörper sind: A76-A/C7 (Maus, IgG1, Epitop: APDTRPAP) und MF06 (Maus, IgG1, Epitop DTRPAP) (siehe: Rye,P.D., Price,M.R., eds., ISOBM TD-4 International Workshop on Monoclonal Antibodies against MUC1, Tumor Biol. 19, Suppl.1, 1998).

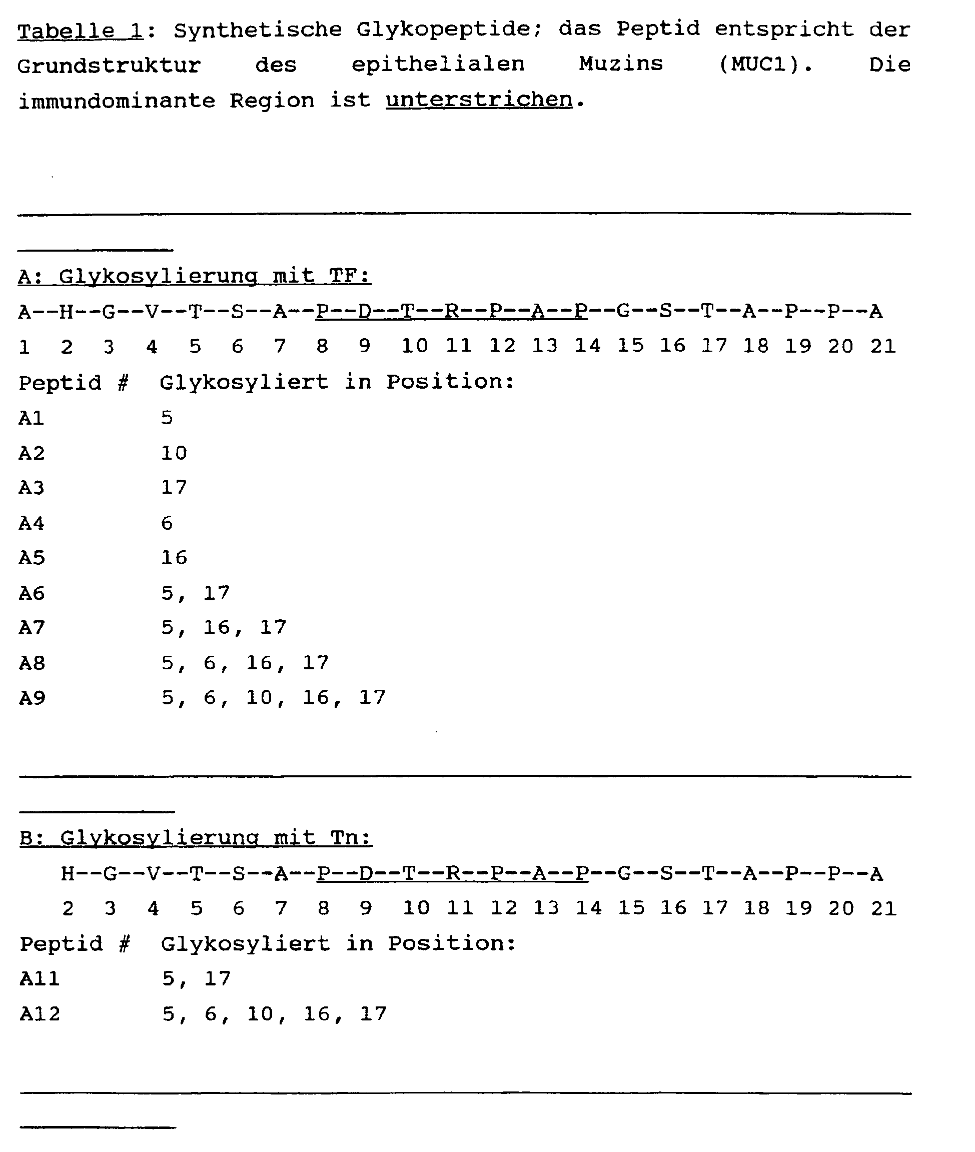

Die Ergebnisse zeigen, daß die in Position 10 glykosylierten Peptide mit den beiden im Beispiel gezeigten Antikörpern signifikant stärker binden als Peptide, die an dieser Stelle nicht glykosyliert sind. Glykosylierungen an anderen Positionen sind ohne Einfluß. Substitution durch Tn oder TF ist gleichwertig. Das in diesem Beispiel demonstrierte Bindungsverhalten wird von anderen MUC1-Antikörpern geteilt; es gibt aber auch Ausnahmen. Die deutlich erhöhte Antigenität der in Position 10 glykosylierten Peptide läßt sich auch in Inhibitionsversuchen zeigen.
Die Ergebnisse zeigen, daß eine Glykosylierung der immundominanten Region des MUC1-Peptids mittels Tn oder TF die Antigenität signifikant erhöht.

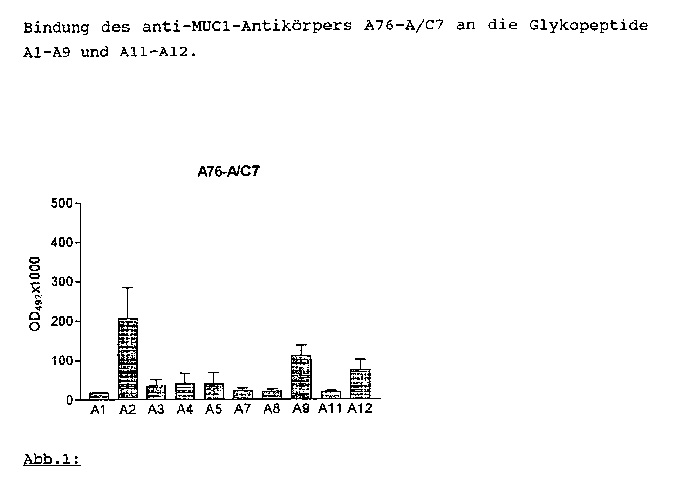

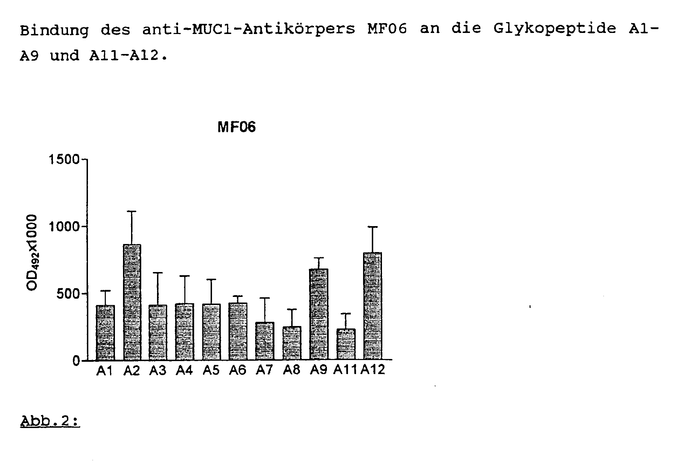

## Patentansprüche

1. Tumorvakzine, enthaltend vom menschlichen epithelialen Muzin MUC1 abgeleitete synthetische Peptide mit der Sequenz PDTRPAP und einer Länge von wenigstens 7 Aminosäuren und maximal 120 Aminosäuren, die am Threonin der enthaltenen immundominanten Regionen PDTRPAP eine O-Glykosylierung mit dem Monosaccharid GalNAc oder mit einem kurzkettigen Oligosaccharid aufweisen, **dadurch gekennzeichnet, dass** die Glykosylierung durch α-D-GalNAc erfolgt ist.

2. Tumorvakzine nach Anspruch 1, **dadurch gekennzeichnet, dass** die synthetischen Peptide mindestens die immundominante Region PDTRPAP und gegebenenfalls weitere benachbarte Aminosäuren beinhalten.

3. Tumorvakzine nach Anspruch 1, **dadurch gekennzeichnet, dass** die synthetischen Peptide eine Länge von > 20 Aminosäuren, haben.

4. Tumorvakzine nach Anspruch 2, **dadurch gekennzeichnet, dass** die synthetischen Peptide eine Länge von 40-120 Aminosäuren haben.

5. Tumorvakzine nach Anspruch 1 bis 4, **dadurch gekennzeichnet, dass** die Glykosylierung durch ein Disaccharid erfolgt.

6. Tumorvakzine nach Anspruch 1 bis 3 und 5, **dadurch gekennzeichnet, dass** die Glykosylierung durch β-D-Gal(1-3)α-D-GalNAc erfolgt.

7. Verwendung der Tumorvakzine nach Anspruch 1 bis 6 zur Herstellung eines Mittels für die Bekämpfung von Tumorzellen aus Mamma-, colorectalen oder Pankreas-karzinomen im Sinne einer aktiven spezifischen Immunisierung.

## Claims

1. Tumour vaccine containing synthetic peptides derived from human epithelial mucine MUC1 with the sequence PDTRPAP and a length of no less than 7 amino acids and no more than 120 amino acids manifesting an O-glycosylation with the monosaccharide GalNAc or with a short-chained oligosaccharide on the threonine of the contained immuno-dominant regions PDTRPAP, wherein the glycosylation has taken place as a result of α-D-GalNAc.

2. Tumour vaccine according to Claim 1, wherein die synthetic peptides contain at least the immuno-dominant region PDTRPAP and, if applicable, further neighbouring amino acids.

3. Tumour vaccine according to Claim 1, wherein the synthetic peptides have a length of > 20 amino acids.

4. Tumour vaccine according to Claim 2, wherein die synthetic peptides have a length of 40-120 amino acids.

5. Tumour vaccine according to Claim 1 to 4, wherein the glycosylation is done as a result of a disaccharide.

6. Tumour vaccine according to Claim 1 to 3 and 5, wherein the glycosylation is done as a result of β-D-Gal(1-3)α-D-GalNAc.

7. Use of the tumour vaccine according to Claim 1 to 6 for the production of an agent for treatment of tumour cells from mamma, colorectal or pancreas carcinomas in the sense of an active specific immunisation.

## Revendications

1. Vaccine anti-tumorale, contenant de la peptide synthétique, dérivée de la mucine épithéliale humaine MUC1 avec la séquence PDTRPAP et une longueur de 7 acides aminés au moins et de 120 acides aminés au plus qui présentent une O-glycosylation avec le monosaccharide GalNAc ou avec un oligosaccharide à chaîne courte sur la thréonine des régions immunodominantes contenues PDTRPAP, **se caractérisant par le fait que** la glycosylation se fait par α-D-GalNAc.

2. Vaccine anti-tumorale selon la revendication 1, **se caractérisant par le fait que** les peptides synthétiques contiennent au moins la région immunodominante PDTRPAP et le cas échéant d'autres acides aminés voisins.

3. Vaccine anti-tumorale selon la revendication 1, **se caractérisant par le fait que** les peptides synthétiques ont une longueur > à 20 acides aminés.

4. Vaccine anti-tumorale selon la revendication 2, **se caractérisant par le fait que** les peptides synthétiques ont une longueur de 40 à 120 acides aminés.

5. Vaccine anti-tumorale selon la revendication 1 à 4, **se caractérisant par le fait que** la glycosylation se fait par un disaccharide.

6. Vaccine anti-tumorale selon la revendication 1 à 3 et 5, **se caractérisant par le fait que** la glycosylation se fait par un β-D-Gal(1-3)α-D-GalNAc.

7. Emploi de la vaccine anti-tumorale selon la revendication 1 à 6 pour la fabrication d'un médicament qui permet de lutter contre des cellules tumorales issues de cancers du sein, colorectaux ou du pancréas dans le sens d'une immunisation spécifique active.
